# EUROPEAN PATENT APPLICATION

(11) **EP 1 972 288 A1**
(43) Date of publication of application: **24.09.2008**
(21) Application number: 08102715.3
(22) Date of filing: 18.03.2008
(51) Int. Cl.: A61B 17/32

(54) **Shaver blade with depth markings**

(30) Priority: 19.03.2007 US 895496 P
(71) Applicant: Arthrex, Inc., Naples, Florida 34108-1945 (US)
(72) Inventor: Bernardini, Robert, Baldwin, NY 11510 (US)
(74) Representative: Long, Giorgio

(57) **Abstract**

A surgical cutting instrument (100) with a tubular member (50) provided with a plurality of markings (90) (for example, depth markings) to aid a surgeon in assessing the dimensions (for example, the depth) of the tissue or structure that is cut or shaped. The tubular member may be an outer tubular member of a cutting instrument having a proximal end and a distal end. An inner tubular member having cutting means may be rotatably disposed within the outer tubular member. The cutting means or cutter may be rotatably disposed within the outer tubular member adjacent to an outer cutting aperture (55) and adjacent the plurality of markings.

## Description

### FIELD OF THE INVENTION

The present invention is directed to surgical cutting instruments having relatively movable inner tubular members.

### BACKGROUND OF THE INVENTION

Surgical cutting instruments in which an inner member is rotated within an elongate tubular outer member are known in surgical procedures where access to the surgical site is via a narrow portal or passage. Typically, the tubular outer member has a distal end with an opening defining a cutting port or window. The inner member has a distal end with a cutting tip for engaging bodily tissue via the opening. Proximal ends of the inner and outer members commonly include hubs which attach to a handpiece having a motor for rotating the inner member relative to the outer member. The distal end of the inner member can have various configurations dependent upon the surgical procedure to be performed. Often the inner member is tubular so that the loose tissue resulting from a cutting, resecting or abrading procedure can be aspirated through the lumen of the inner member.

An improved cutting instrument used in performing closed surgery, such as arthroscopic surgery, that can provide information regarding the dimensions of the tissue being cut or shaped during a cutting, resecting or abrading procedure is needed. A method of conducting arthroscopic surgery, wherein the step of cutting (or resecting or abrading) and the step of providing information about the tissue being cut or shaped is performed using the same cutting instrument, is also needed.

### SUMMARY OF THE INVENTION

The present invention provides a surgical cutting instrument comprising a tubular member provided with a plurality of markings (for example, depth markings) to aid a surgeon in assessing the dimensions (for example, the depth) of the tissue or structure that is cut or shaped. The tubular member may be part of a surgical arthroscopic cutting instrument. The present invention also provides a method of conducting an arthroscopic surgical procedure wherein the step of cutting, resecting, or abrading, and the step of providing information about the tissue being cut or shaped are performed using the same cutting instrument.

### BRIEF DESCRIPTION OF THE DRAWINGS

Other features and advantages of the present invention will become apparent when the following detailed description is read in conjunction with the accompanying drawings, in which:

Figure 1 is a cross-sectional view of a surgical cutting instrument provided with depth markings according to an exemplary embodiment of the present invention.

Figure 2 is a top view of the surgical cutting instrument of Figure 1.

Figure 3 is a side view of the surgical cutting instrument of Figure 2 and illustrating the depth markings.

### DETAILED DESCRIPTION OF THE PREFERRED EMBODIMENTS

The following description is provided to enable any person skilled in the art to make and use the invention and sets forth the best modes contemplated by the inventors of carrying out their invention. Various modifications, however, will remain readily apparent to those skilled in the art.

The present invention provides a surgical cutting instrument comprising a tubular member provided with a plurality of markings (for example, depth markings) to aid a surgeon in assessing the dimensions (for example, the depth) of the tissue or structure that is cut or shaped.

According to an exemplary embodiment, the tubular member is an outer tubular member of a cutting instrument having a proximal end and a distal end. An inner tubular member includes a proximal end portion having cutting means (or cutter), and a distal end portion. The inner tubular member is rotatably disposed within the outer tubular member. The cutting means or cutter is rotatably disposed within the outer tubular member adjacent to an outer cutting aperture and adjacent the plurality of markings.

The present invention also provides a method of conducting an arthroscopic surgical procedure using a surgical cutting instrument provided with depth markings. The method comprises the steps of: (i) providing a surgical cutting instrument having an outer tubular member with a plurality of markings, for example, depth markings; (ii) positioning the surgical cutting instrument in the proximity of a surgical site undergoing an arthroscopic procedure; and (iii) employing the surgical cutting instrument to conduct the arthroscopic procedure.

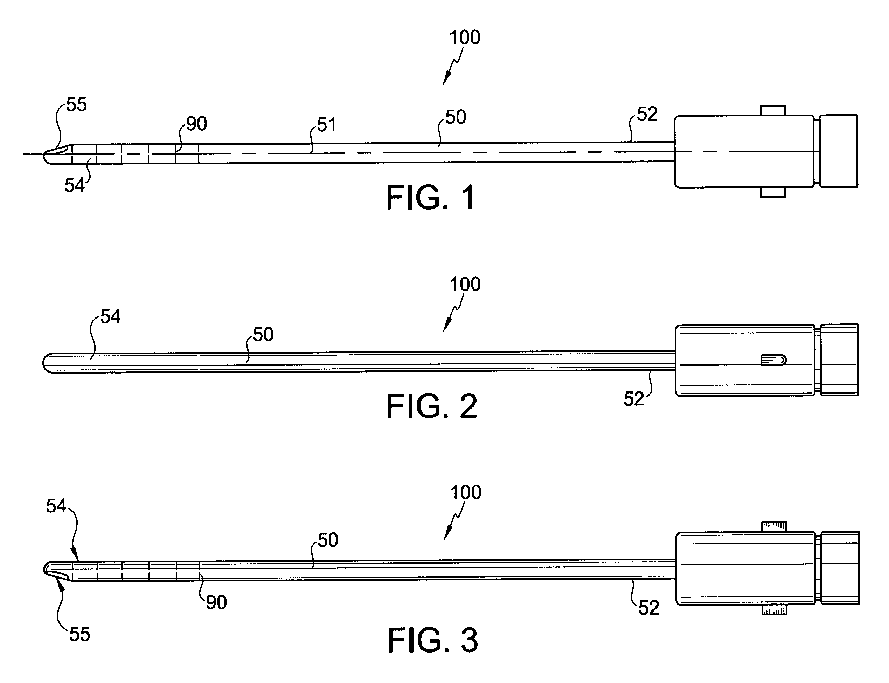

Figures 1-3 illustrate an exemplary embodiment of a surgical cutting instrument 100 of the present invention, which is provided with an outer tubular member having a plurality of markings (for example, depth markings) to aid a surgeon in assessing the dimensions (for example, the shape or depth) of the tissue or structure that is being cut or shaped with the cutting instrument.

As shown in the drawings, surgical cutting instrument 100 comprises an elongate tubular member 50 having a proximal end 52 and a distal end 54. An inner tubular member (not shown) includes a proximal end portion having cutting means (not shown), and a distal end portion. The inner tubular member may be rotatably disposed within the tubular member 50. The cutting means or cutter may be rotatably disposed within the tubular member 50 adjacent to an outer cutting aperture or window 55.

In exemplary embodiments, the cutting aperture 55 may be provided with a first edge and a second edge (for example, a first lateral edge and a second lateral edge) each of the edges being provided with a plurality of cutting teeth having various geometries, for example, a pyramidal configuration. The plurality of teeth may be positioned along the lateral cutting edges of the window 55, and they may be configured to allow easy penetration into tissue. The cutting edges may be formed by laser cutting technology, for example. The teeth of each edge may be symmetrically disposed relative to the tube axis when viewed in a plan view. In another embodiment, the teeth may be asymmetrically disposed. In other embodiments, the cutting window 55 may be symmetrically or asymmetrically disposed relative to the tube axis when viewed in a plan view. The tubular member 50 may be formed from a medically acceptable material such as stainless steel, and may have a cylindrical or ellipsoidal cross-section, among others.

As shown in Figure 3, a plurality of markings 90 are provided at the distal end 54 of the tubular member 50. Markings 90 aid a surgeon in assessing the dimensions (for example, the depth) of the cut or incision performed during arthroscopic surgery with the surgical cutting instrument 100. Markings 90 may be laser sliced or laser cut, for example, or may be formed by other known techniques in the art. As illustrated in Figure 3, markings 90 may be provided only on one side of the outer surface of the tubular member 50, for example, only on one lateral side or on two lateral sides of the tubular member 50. Markings 90 may comprise, for example, a plurality of parallel lines that are about perpendicular to longitudinal axis 51 (Figure 1) of the tubular member 50. Markings 90 may be provided, however, in other geometries and configurations, and in directions which are not about perpendicular to the longitudinal axis of the cutting instrument, depending on the characteristics of the arthroscopic surgery involved, and as desired.

An inner tubular member may be disposed coaxially or concentrically within the outer tube 50, so that the cutting means of the inner tubular member is disposed at a distal end of the inner tube. The inner and outer tubes may be formed from a medically acceptable material such as stainless steel, and may have a cylindrical or ellipsoidal cross-section, among others. Preferably, the inner member is tubular so that the loose tissue resulting from a cutting, resecting or abrading procedure can be aspirated through the lumen of the inner member. The proximal ends of the inner and outer members may include hubs which attach to a handpiece having a motor for rotating the inner member relative to the outer member 50.

The cutting instrument or shaver 100 with a plurality of markings described above may be part of an arthroscopic shaver employed in various surgical medical procedures such as conventional open surgeries or in other, less invasive, techniques that use cannulas or various port access devices. The present invention has applications in surgical procedures where the target tissue is ablated or shaped, and may be employed in cutting various body parts such as the knee, shoulder, hip, ankle, elbow, hand or foot. For example, the tubular member 100 of the present invention may be part of an arthroscopic shaver employed in arthroscopic surgery of a knee joint or hip structure.

Although the present invention has been described in connection with preferred embodiments, many modifications and variations will become apparent to those skilled in the art. While preferred embodiments of the invention have been described and illustrated above, it should be understood that these are exemplary of the invention and are not to be considered as limiting. Accordingly, it is not intended that the present invention be limited to the illustrated embodiments, but only by the appended claims.

## Claims

1. An arthroscopic cutting instrument, comprising:
an outer tubular member comprising a distal end, a proximal end, an inner cylindrical surface, an outer cylindrical surface, a cutting window located at the distal end for receiving anatomical tissue therethrough, and a plurality of markings provided on the outer cylindrical surface and adjacent the distal opening; and
an inner tubular member rotatably disposed within the outer tubular member, the inner tubular member comprising cutting means disposed at a most distal end of the inner tubular member.

2. The arthroscopic cutting instrument of claim 1, wherein the cutting means is a shaver blade or an abrader.

3. The arthroscopic cutting instrument of claim 1, wherein at least one of the plurality of markings is formed by laser slicing or laser cutting.

4. The arthroscopic cutting instrument of claim 1, wherein the plurality of markings extend in a direction about perpendicular to the longitudinal axis of the outer tubular member.

5. The arthroscopic cutting instrument of claim 1, wherein the cutting window is in communication with the cutting means of the inner tubular member.

6. The arthroscopic cutting instrument of claim 1, wherein the cutting window is provided with a first lateral cutting edge and a second lateral cutting edge.

7. The arthroscopic cutting instrument of claim 6, wherein the cutting window is provided with a first plurality of teeth located on the first lateral cutting edge, and a second plurality of teeth located on the second lateral cutting edge.

8. The arthroscopic cutting instrument of claim 7, wherein the first and second plurality of teeth are asymmetrically located relative to the longitudinal axis of the outer tubular member.

9. The arthroscopic cutting instrument of claim 7, wherein the first and second plurality of teeth are symmetrically located relative to the longitudinal axis of the outer tubular member.

10. The arthroscopic cutting instrument of claim 7, wherein the first and second plurality of teeth have a pyramidal geometry.
